# EUROPÄISCHE PATENTANMELDUNG

(11) **EP 3 865 499 A1**
(43) Veröffentlichungstag der Anmeldung: **18.08.2021**
(21) Anmeldenummer: 20156797.1
(22) Anmeldetag: 12.02.2020
(51) Int. Cl.: C07H 3/02, C07H 1/00, C07H 1/06

(54) **ALLULOSEKONZENTRATE IN FESTER AMORPHER FORM**

(71) Anmelder: Savanna Ingredients GmbH, 50189 Elsdorf (DE)
(72) Erfinder: JUNKLEWITZ, Anna, 42119 Wuppertal (DE); MOHR, Stephan, 53879 Euskirchen (DE); BUTZ, Steffen, 52372 Kreuzau (DE); BOGNAR, Michael, 41540 Dormagen (DE); KOCH, Timo, 50189 Elsdorf (DE)
(74) Vertreter: Fabry, Bernd

(57) **Zusammenfassung**

Vorgeschlagen werden Allulosekonzentrate in fester amorpher Form, welche sich dadurch auszeichnen, dass sie einen Gehalt von mindestens etwa 20 Gew.-% Allulose aufweisen sowie ein Verfahren zu deren Herstellung.

## Beschreibung

### GEBIET DER ERFINDUNG

Die Erfindung befindet sich auf dem Gebiet der Lebensmitteltechnologie und betrifft Allulosekonzentrate sowie ein Verfahren zu deren Herstellung.

### TECHNOLOGISCHER HINTERGRUND

Allulose (Psicose) ist ein kalorienarmer Zucker mit ähnlich süßem Geschmack von Zucker. Allulose ist einer von vielen verschiedenen Zuckern, die in der Natur in sehr geringen Mengen vorkommen. Allulose wurde ursprünglich aus Weizen identifiziert und wurde seitdem in bestimmten Früchten wie Jackfrucht, Feigen und Rosinen gefunden. Allulose ist natürlich in kleinen Mengen in einer Vielzahl von süßen Lebensmitteln wie Karamellsauce, Ahornsirup und braunem Zucker enthalten. Allulose wird vom Körper absorbiert, aber nicht metabolisiert, sodass sie nahezu kalorienfrei ist.

Aufgrund des wachsenden Interesses eines großen Teils der Bevölkerung an *"gesunder Ernährung"* und gesunder Lebensweise allgemein, hat Allulose als kalorienfreier Zucker ein großes Interesse in der Lebensmittelindustrie und in der wissenschaftlichen Community erweckt.

In der Regel wird Allulose in Form eines Sirups kommerzialisiert. Jedoch zeigen diese Allulosesirupe erhebliche Stabilitätsprobleme, so dass die Qualität des Produktes nach einer längeren Lagerung, insbesondere in Hinblick auf die Sensorik, Farbe, Reinheit und Temperaturbeständigkeit nicht mehr optimal ist.

Das Trocknen von Lebensmitteln gehört zu den ältesten und zugleich natürlichsten Konservierungsmethoden und hat sich als schonende Haltbarmachung bis heute bewährt.

Unter Trocknung versteht man allgemein den Entzug von Flüssigkeiten aus dem Trocknungsgut, durch Verdunstung, Verdampfung oder andere technische Verfahren. Das Trockengut kann in Form von Pulver oder Granulaten, in Stücken oder Scheiben erstellt oder, z.B. bei Früchten, im Ganzen belassen werden. Durch den Feuchtigkeitsentzug weist das Trockengut nach der Behandlung ein wesentlich geringeres Gewicht auf, bei gleichzeitiger Beibehaltung der wichtigen und wertvollen Bestandteile.

Die Gefriertrocknung ist ein besonders bevorzugtes Trocknungsverfahren, da sie sich als ein besonders schonendes Trocknungsverfahren in Bezug auf Geschmack oder Rehydrationsfähigkeit bewiesen hat. Bei diesem Verfahren muss das Trocknungsgut jedoch erst eingefroren werden, um daraus das Wasser zu sublimieren. Nachteil dieses Verfahren ist die Tatsache, dass es sehr energieaufwendig ist.

### RELEVANTER STAND DER TECHNIK

Verschiedene Verfahren zur Herstellung von Allulose sind aus dem Stand der Technik bekannt, wie beispielsweise aus den Dokumenten WO 2018/087261A1(Pfeifer & Langen GmbH & Co. KG) oder WO 2016/135358 A1(Tate & Lyle Technology Limited).

### AUFGABE DER ERFINDUNG

Die Aufgabe der vorliegenden Erfindung hat somit darin bestanden, Allulosekonzentrate in fester, amorpher Form, die aus einem Allulosesirup hergestellt werden, zur Verfügung zu stellen, die frei von den eingangs geschilderten Nachteilen sind. Insbesondere sollten die neuen Allulosekonzentrate im Vergleich zu Allulose in Sirupform eine verbesserte Lager- und Temperaturbeständigkeit aufweisen, so dass ein Allulosesirup nach Rekonstitution durch Zugabe von Wasser erhalten wird, welcher in Hinblick auf seine Zusammensetzung und organoleptischen Eigenschaften von dem frisch hergestellten, ursprünglichen Allulosesirup nicht zu unterscheiden ist.

Gleichzeitig hat auch eine Aufgabe der vorliegenden Erfindung darin bestanden, ein energieschonendes Verfahren zur Verfügung zu stellen, das die Herstellung der oben genannten festen amorphen Allulosekonzentrate ermöglicht.

### BESCHREIBUNG DER ERFINDUNG

Gegenstand der vorliegenden Erfindung sind Allulosekonzentrate in fester amorpher Form, die sich dadurch auszeichnen, dass sie einen Gehalt von mindestens etwa 20 Gew.-% Allulose aufweisen.

Überraschenderweise wurde gefunden, dass die erfindungsgemäßen Allulosekonzentrate eine erheblich verbesserte Lager- und Temperaturstabilität im Vergleich zu den entsprechenden Allulosen in Sirupform aufweisen, aus denen sie gewonnen wurden.

Ferner wurde überraschenderweise gefunden, dass nach einer Lagerzeit von über einem Jahr, die erfindungsgemäßen festen amorphen Allulosekonzentrate die Herstellung eines Allulosesirups durch Rekonstitution mittels Zugabe von Wasser ermöglichen, der im Hinblick auf seine Zusammensetzung und organoleptischen Eigenschaften von dem frisch hergestellten ursprünglichen Allulosesirup, aus dem dieser gewonnen wurde, nicht zu unterscheiden war.

Im Sinne der vorliegenden Erfindung bezieht sich der Begriff *"Konzentrat"* auf trockene oder wässrige Zusammensetzungen, in denen eine Gesamtmenge an Allulose von 20 bis 99 Gew.-%, bezogen auf die Gesamtfeststoffe, enthalten ist.

Unter *"Sirup"* im Sinne der vorliegenden Erfindung ist eine dickflüssige, konzentrierte Lösung eines Zuckers oder Zuckeraustauschstoffs zu verstehen, die durch Kochen oder andere Techniken, insbesondere durch enzymatische Spaltungs- oder Umwandlungsprozesse, aus zuckerhaltigen Flüssigkeiten oder Pflanzenextrakten gewonnen wird.

Allulose, auch Psicose genannt, ist eine Ketohexose. Für die Zwecke der vorliegenden Erfindung wird Allulose vorzugsweise in Form des D-Enantiomers bereitgestellt, d.h. D-Allulose (CAS-Nr. 551-68-8). D-Allulose kann in Form von verschiedenen Anomeren, wie z.B. α -D-Allulose und β-D-Allulose, vorhanden sein.

In einer bevorzugten Ausführungsform liegen die erfindungsgemäßen festen amorphen Allulosekonzentrate zu mindestens 90 Gew.-%, vorzugsweise zu mindestens 95 Gew.-% und insbesondere zu etwa 98 bis etwa 99,5 Gew.-% in amorpher Form vor.

In einer bevorzugten Ausführungsform weisen die erfindungsgemäßen Allulosekonzentrate einen Gehalt von mindestens etwa 40 Gew.-% Allulose auf, vorzugsweise mindestens etwa 50 Gew.-%, vorzugsweise mindestens etwa 60 Gew.-%, insbesondere bevorzugt mindestens etwa 70 Gew.-%, und insbesondere bevorzugt mindestens etwa 75 Gew.-%.

Die erfindungsgemäßen Allulosekonzentrate der vorliegenden Erfindung zeichnen sich dadurch aus, dass der Gehalt an Allulose im Bereich von etwa 20 bis etwa 99 Gew.-% liegt.

In einer bevorzugten Ausführungsform der erfindungsgemäßen Allulosekonzentrate liegt der Gehalt an Allulose im Bereich von etwa 25 bis etwa 95 Gew.-%, vorzugsweise im Bereich von etwa 40 bis etwa 90 Gew.-%, bevorzugt im Bereich von etwa 45 bis etwa 85 Gew.-%, und insbesondere bevorzugt im Bereich von etwa 50 bis etwa 80 Gew.-%.

Darüber hinaus können die erfindungsgemäßen Allulosekonzentrate auch Fructose enthalten. Die erfindungsgemäßen Allulosekonzentrate können dabei einen Gehalt von maximal etwa 80 Gew.-% Fructose aufweisen.

In einer weiteren bevorzugten Ausführungsform weisen die erfindungsgemäßen Allulosekonzentrate einen Gehalt von maximal etwa 60 Gew.-%, vorzugsweise maximal etwa 50 Gew.-%, vorzugsweise maximal etwa 40 Gew.-%, insbesondere bevorzugt maximal etwa 30 Gew.-%, und insbesondere bevorzugt maximal etwa 25 Gew.-% Fructose auf.

In einer bevorzugten Ausführungsform der erfindungsgemäßen Allulosekonzentrate liegt der Gehalt an Fructose im Bereich von etwa 25 bis etwa 80 Gew.-%, vorzugsweise im Bereich von etwa 40 bis etwa 60 Gew.-%, und insbesondere bevorzugt im Bereich von etwa 45 bis etwa 55 Gew.-%.

In einer weiteren bevorzugten Ausführungsform weisen die erfindungsgemäßen Allulosekonzentrate einen Gehalt von mindestens etwa 20 Gew.-% Allulose und maximal etwa 80 Gew.-% Fructose auf, vorzugsweise mindestens etwa 25 Gew.-% Allulose und maximal etwa 75 Gew.-% Fructose, vorzugsweise mindestens etwa 40 Gew.-% Allulose und maximal etwa 60 Gew.-% Fructose, vorzugsweise mindestens etwa 50 Gew.-% Allulose und maximal etwa 50 Gew.-% Fructose, vorzugsweise mindestens etwa 60 Gew.-% Allulose und maximal etwa 40 Gew.-% Fructose, bevorzugt mindestens etwa 70 Gew.-% Allulose und maximal etwa 30 Gew.-% Fructose, und insbesondere bevorzugt mindestens etwa 75 Gew.-% Allulose und maximal etwa 25 Gew.-% Fructose auf.

In einer ersten bevorzugten Ausführungsform beträgt das Gewichtsverhältnis Allulose:Fructose etwa 20:80 bis etwa 80:20, in einer zweiten Ausführungsform etwa 90:10 bis 95:5.

Die erfindungsgemäßen festen amorphen Allulosekonzentrate zeichnen sich dadurch aus, dass sie einen Gehalt von maximal etwa 0,1 Gew.-% Wasser aufweisen, vorzugsweise von maximal etwa 0,05 Gew.-% und besonders bevorzugt von maximal etwa 0,01 Gew.-%.

Ein weiterer Gegenstand der Erfindung betrifft ein Verfahren zur Herstellung eines festen amorphen Allulosekonzentrates, umfassend oder bestehend aus folgenden Schritten:
(a) Bereitstellung eines Allulosekonzentrates in Sirupform,
(b) optional Aufkonzentrierung des Produktes aus Schritt (a),
(c) Trocknung des Zwischenproduktes aus Schritt (b) oder des Allulosekonzentrates aus Schritt (a) auf einem Sprüh-, Gefrier- oder Bandtrockner mit anschließender Zerkleinerung, und schließlich
(d) Ausschleusen und gegebenenfalls nachfolgende Verpackung des trockenen Endproduktes.

Im ersten Abschnitt des Verfahrens erfolgt zunächst die Bereitstellung eines Allulosekonzentrates in Sirupform; es kann sich dabei um ein kommerziell erhältliches Allulosekonzentrat in Sirupform bzw. um ein Allulosekonzentrat in Sirupform handeln, welches nach einem Verfahren aus dem Stand der Technik Verfahren hergestellt wurde.

In einer bevorzugten Ausführungsform umfasst oder besteht das Allulosekonzentrat in Sirupform aus Schritt (a) aus folgenden Komponenten:
(i) etwa 90 bis etwa 95 Gew.-% Allulose,
(ii) etwa 0 bis etwa 10 Gew.-% Fructose,
(iii) 0 bis etwa 5 Gew.-% weitere Kohlenhydrate, verschieden von Allulose und Fructose, mit der Maßgabe, dass sich die Mengenangaben zu 100 Gew.-% ergänzen. Der guten Ordnung halber sei darauf hingewiesen, dass Ausführungsformen, die sich zu mehr oder zu weniger als 100 Gew.-% ergeben, weder von der Erfindung eingeschlossen noch von den Ansprüchen umfasst werden. Der Fachmann ist in jedem Fall in der Lage, nach Maßgabe von Beschreibung und Beispielen solche Zusammensetzungen aufzufinden, die die technische Lehre erfüllen, ohne dazu erfinderisch tätig werden zu müssen.

In einer bevorzugten Ausführungsform weist das Allulosekonzentrat in Sirupform aus Schritt (a) einen Gehalt von etwa 25 bis etwa 40 Gew.-% Wasser auf, insbesondere bevorzugt einen Gehalt von etwa 30 Gew.-%.

In einer weiteren bevorzugten Ausführungsform weist das Allulosekonzentrat in Sirupform aus Schritt (a) eine Trockenmasse von etwa 10 bis etwa 30 Gew.-% auf.

An dieser Stelle sei aber auch erwähnt, dass es für die Herstellung der festen amorphen Allulosekonzentrate, die eine Reinheit von praktisch 100 % aufweisen und praktisch frei von Fructose bzw. von weiteren Kohlenhydraten (verschieden von Allulose und Fructose) sein sollen, vorteilhaft ist, wenn eine Kristallisation der Allulose aus dem Allulosekonzentrat in Sirupform aus Schritt (a) erfolgt.

Dabei wird das Allulosekonzentrat in Sirupform aus Schritt (a) einer Verdampfungs-, Kühlungs- oder Verdrängungskristallisation mit einem protischen Lösungsmittel oder einer Kombination aus diesen unterworfen.

Die so gewonnenen Allulosekristalle werden vorzugsweise durch Filtration abgetrennt, und dann einer Trocknung mit nachfolgender Zerkleinerung unterworfen; anschließend wird das so erhaltene trockene Endprodukt ausgeschleust (die Schritte (c) und (d) des erfindungsgemäßen Verfahrens).

Als protische Lösungsmittel kommen für die Kristallisation der Allulose aus dem Allulosekonzentrat in Sirupform verzweigte oder unverzweigte C₁-C₆-Alkohole, vorzugsweise Ethanol und/oder Isopropanol in Frage. Bei der Kristallisation besteht der Feststoff dann ganz oder zumindest ganz überwiegend aus β-D-Psicopyranose.

Wie bereits erwähnt, kann optional das Allulosekonzentrat in Sirupform aus Schritt (a) in einen Eindampfer, vorzugsweise einen Plattensteigfilmverdampfer eingespeist werden, in dem es unter Anlegen eines Temperaturgradienten von etwa 100 bis auf etwa 50 °C und insbesondere etwa 95 bis auf etwa 55 °C und/oder eines Druckgradienten schonend entwässert wird, so dass das so gewonnene Zwischenprodukt mit einer Trockenmasse von etwa 60 bis etwa 85 Gew.-%, vorzugsweise etwa 80 Gew.-% anfällt. Das Allulosekonzentrat in Sirupform aus Schritt (a) bzw. das konzentrierte Zwischenprodukt aus Schritt (b) wird dann ebenfalls getrocknet.

Das oben beschriebene Verfahren ermöglicht die Herstellung der festen amorphen Allulosekonzentrate. Daher betrifft ein weiterer Gegenstand der vorliegenden Erfindung amorphe, feste Allulosekonzentrate, die dadurch erhältlich sind, dass man
(a) ein Allulosekonzentrat in Sirupform bereitstellt,
(b) optional das Konzentrat aus Schritt (a) aufkonzentriert,
(c) das Zwischenprodukt aus Schritt (b) oder das Allulosekonzentrat aus Schritt (a) einer Trocknung auf einem Sprüh-, Gefrier- oder Bandtrockner mit anschließender Zerkleinerung unterwirft, und schließlich
(d) das trockene Endprodukt ausschleust und gegebenenfalls verpackt.

Bezüglich der technischen Aspekte des Herstellungsverfahrens gelten die vorstehenden Ausführungen mit, so dass sich eine Wiederholung erübrigt.

Zusammenfassend lässt sich feststellen, dass die erfindungsgemäßen festen amorphen Allulosekonzentrate eine ausgezeichnete Alternative zur Lagerung über einen längeren Zeitraum der bisher üblichen Allulosesirupe darstellen, da sich die Konzentrate durch eine verbesserte Beständigkeit und Stabilität auszeichnen.

Darüber hinaus lassen sich aus den erfindungsgemäßen festen amorphen Allulosekonzentraten Allulosesirupe in einer sehr einfachen und effizienten Art und Weise reproduzierbar herstellen, wie in dem Beispiel der vorliegenden Erfindung gezeigt wird.

### BEISPIELE

Die vorliegende Erfindung wird unter Bezugnahme auf die nachstehenden Beispiele leichter zu verstehen sein. Jedoch dienen diese Beispiele lediglich zur Veranschaulichung der Erfindung und können nicht als einschränkend in Bezug auf den Schutzbereich der Erfindung ausgelegt werden.

### BEISPIEL 1

Ein frisch hergestelltes Allulosekonzentrat in Sirupform **(Produkt A)** mit einer Trockenmasse von 70 Gew.-% (65 Gew.-% Allulose und 5 Gew.-% Fructose) wurde in einem Plattensteigfilmverdampfer unter Anlegen eines Temperaturgradienten von 95 auf 55 °C bis zu einer Trockenmasse von 75 Gew.-% eingedampft. Das erhaltene Zwischenprodukt wurde über eine Förderpumpe auf das Förderband eines Vakuumbandtrockners aufgegeben. Dieser wies drei Heizzonen (60, 50 und 40 °C) und eine Kühlzone (20 °C) auf (Druck: 40 mbar). Das erhaltene Produkt wurde zerkleinert und verpackt, und für 14 Monate bei 22 °C gelagert. Nach dieser Zeit wurde der Sirup durch die Zugabe einer entsprechenden Wassermenge rekonstituiert, so dass ein Allulosekonzentrat in Sirupform **(Produkt A1)** erhalten wurde, welches was die Zusammensetzung anbelangte zum ursprünglichen **Produkt A** identisch war.

### VERGLEICHSBEISPIEL V1

Eine Glasflasche wurde mit einem frisch hergestellten **Produkt A,** wie im Beispiel 1 definiert, befüllt. Das Produkt wurde für 14 Monate unter identischen Bedingungen wie das erfindungsgemäße Produkt gemäß Beispiel 1 gelagert, so dass ein **Produkt A2** erhalten wurde.

### EVALUIERUNG DER PRODUKTE A, A1 UND A2

Der frisch hergestellte Sirup **(Produkt A)**, der frisch aus dem erfindungsgemäßen Allulosekonzentrat (nach 14 Monaten Lagerung) hergestellte Sirup **(Produkt A1**) und das **Produkt A2** (Produkt A nach 14 Monaten Lagerung) wurden von einem Panel bestehend aus fünf erfahrenen und geschulten Testern evaluiert (3 = ausgeprägt, 2 = vorhanden, 1 = nicht festzustellen). Dabei wurden folgende Parameter evaluiert:
- Optik (Bräunung)
- Geschmack (bitter und metallischer Eindruck)

Die Ergebnisse sind in **Tabelle 1** zusammengefasst.

**Tabelle 1**

| Produktbewertung | | |
|---|---|---|
| **Produkte** | **Optik** | **Geschmack** |
| **Produkt A** | 1 | 1 |
| **Produkt A1** | 1 | 1 |
| **Produkt A2** | 3 | 3 |

Die Daten der Tabelle 1 zeigen eindeutig, dass eine 14-monatige Lagerung der erfindungsgemäßen festen amorphen Allulosekonzentrate keinen Einfluss auf die Qualität des daraus hergestellten Sirups hat. Die erfindungsgemäßen Konzentrate zeigen eine ausgezeichnete Löslichkeit, so dass die Rekonstitution eines Sirups durch Zugabe von einer entsprechenden Menge von Wasser bei Raumtemperatur und mittels üblichen Rührens problemlos erfolgt.

Das **Produkt A2** zeigte hingegen aufgrund der Instabilität des Sirups eine erhebliche Verschlechterung sowohl der optischen als auch der organoleptischen Eigenschaften,

Die erfindungsgemäßen festen amorphen Allulosekonzentrate stellen daher eine ausgezeichnete Alternative für die Lagerung der bisher üblichen Allulosesirupe über einen längeren Zeitraum dar, da die Konzentrate sich durch ihre Beständigkeit und Stabilität auszeichnen. Darüber hinaus lassen sich aus den erfindungsgemäßen festen amorphen Allulosekonzentraten Allulosesirupe in einer sehr einfachen und effizienten Art und Weise reproduzierbar herstellen.

## Patentansprüche

1. Allulosekonzentrat in fester amorpher Form, **dadurch gekennzeichnet, dass** das Konzentrat einen Gehalt von mindestens etwa 20 Gew.-% Allulose aufweist.

2. Allulosekonzentrat nach Anspruch 1, **dadurch gekennzeichnet, dass** das Konzentrat einen Gehalt von maximal etwa 80 Gew.-% Fructose aufweist.

3. Allulosekonzentrat nach den Ansprüchen 1 und/oder 2, **dadurch gekennzeichnet, dass** das Konzentrat einen Gehalt von mindestens etwa 80 Gew.-% Allulose und maximal etwa 20 Gew.-% Fructose aufweist.

4. Allulosekonzentrat nach mindestens einem der vorangehenden Ansprüche 1 bis 3, **dadurch gekennzeichnet, dass** das Gewichtsverhältnis Allulose: Fructose etwa 20:80 bis etwa 80:20 beträgt.

5. Allulosekonzentrat nach mindestens einem der vorangehenden Ansprüche 1 bis 4, **dadurch gekennzeichnet, dass** das Konzentrat einen Gehalt von maximal etwa 0,1 Gew.-% Wasser aufweist.

6. Verfahren zur Herstellung eines Allulosekonzentrates in fester amorpher Form, umfassend oder bestehend aus folgenden Schritten:
(a) Bereitstellung eines Allulosekonzentrates in Sirupform,
(b) optional Aufkonzentrierung des Produktes aus Schritt (a),
(c) Trocknung des Zwischenproduktes aus Schritt (b) oder des Allulosekonzentrates aus Schritt (a) auf einem Sprüh-, Gefrier- oder Bandtrockner mit anschließender Zerkleinerung, und schließlich
(d) Ausschleusen und gegebenenfalls nachfolgende Verpackung unter Vakuum des trockenen Endproduktes.

7. Verfahren nach Anspruch 6 **dadurch gekennzeichnet, dass** das Allulosekonzentrat in Sirupform aus Schritt (a) folgende Komponenten umfasst oder aus folgenden Komponenten besteht:
(i) etwa 90 bis etwa 95 Gew.-% Allulose,
(ii) etwa 5 bis etwa 10 Gew.-% Fructose,
(iii) 0 bis etwa 5 Gew.-% weitere Kohlenhydrate, verschieden von Allulose und Fructose,
mit der Maßgabe, dass sich die Mengenangaben zu 100 Gew.-% ergänzen.

8. Verfahren nach mindestens einem der Ansprüche 6 und/oder 7, **dadurch gekennzeichnet, dass** das Allulosekonzentrat in Sirupform aus Schritt (a) einen Gehalt von etwa 5 bis etwa 40 Gew.-% Wasser aufweist.

9. Verfahren nach mindestens einem der Ansprüche 6 bis 8, **dadurch gekennzeichnet, dass** das Allulosekonzentrat in Sirupform aus Schritt (a) einen Gehalt von etwa 25 bis etwa 30 Gew.-% Wasser aufweist.

10. Verfahren nach mindestens einem der Ansprüche 6 bis 9, **dadurch gekennzeichnet, dass** das Allulosekonzentrat in Sirupform aus Schritt (a) eine Trockenmasse von etwa 10 bis etwa 30 Gew.-% aufweist.

11. Verfahren nach mindestens einem der Ansprüche 6 bis 10, **dadurch gekennzeichnet, dass** das Zwischenprodukt aus Schritt (b) eine Trockenmasse von etwa 40 bis etwa 80 Gew.-% aufweist.

12. Verfahren nach mindestens einem der Ansprüche 6 bis 11, **dadurch gekennzeichnet, dass** das Zwischenprodukt aus Schritt (b) eine Trockenmasse von etwa 75 Gew.-% aufweist.

13. Verfahren nach mindestens einem der Ansprüche 6 bis 12, **dadurch gekennzeichnet, dass** man die Trocknung bei Temperaturen im Bereich von etwa 60 bis etwa 130 °C und einem Druck von etwa 15 bis etwa 40 mbar durchführt.
